# EUROPEAN PATENT APPLICATION

(11) **EP 4 523 743 A1**
(43) Date of publication of application: **19.03.2025**
(21) Application number: 23197940.2
(22) Date of filing: 18.09.2023
(51) Int. Cl.: A61N 7/00, A61B 17/225

(54) **ULTRASOUND THERAPY DEVICE**

(71) Applicant: NV Gymna Uniphy, 3740 Bilzen (BE)
(72) Inventor: JASPERS, Jochen, 3740 Bilzen (BE); REMANS, Ivo, 3740 Bilzen (BE)
(74) Representative: Gevers Patents

(57) **Abstract**

The invention provides and ultrasound therapy device (100) comprising an ultrasound applicator (300), a motion detection system (340) and a processing unit (210). The ultrasound applicator (300) comprises ultrasound generating means (324) configured for generating ultrasound. The ultrasound applicator (300) is configured for being held and moved by a user. The motion detection system (340) is configured for detecting motion of the ultrasound applicator (300). The processing unit (210) is operationally connected to the motion detection system (340) and configured for receiving motion detection data from the motion detection system (340). The processing unit (210) is configured for determining a translational velocity of the ultrasound applicator (300) from the motion detection data, and for generating at least one feedback signal based on the translational velocity of the ultrasound applicator (300).

## Description

### Technical field

The present invention relates to an ultrasound therapy device. Furthermore, the present invention also relates to a computer implemented method for operating an ultrasound therapy device.

### Background art

From the prior art there is known an ultrasound therapy device for medical treatment performed on the human body using therapeutic ultrasound technology. The ultrasound therapy device comprises a control device configured for a user to view and change settings of the ultrasound therapy device, such as for example treatment parameters. The ultrasound therapy device comprises an ultrasound applicator. The ultrasound applicator comprises ultrasound generating means configured for generating ultrasound, i.e. ultrasound to be applied to a patient. The ultrasound applicator is configured for being held and moved by a user, i.e. for the purpose of applying the generated ultrasound to the patient while the ultrasound applicator is moved over the skin of the patient. The ultrasound generating means are arranged in a cup portion of the ultrasound applicator. The cup portion of the ultrasound applicator is connected in a fixed manner to a main body of the ultrasound applicator.

The ultrasound therapy device known from the prior art has the disadvantage that therapeutic ultrasound treatment with the ultrasound therapy device requires precise movement and speed control of the ultrasound applicator for optimal results and patient safety. Moving too fast can render the treatment ineffective, while moving too slow can lead to adverse effects such as internal burns.

The ultrasound therapy device known from the prior art also has the disadvantage that if a user wants to set the intensity of the ultrasound generated by the ultrasound generating means of the ultrasound applicator while moving the ultrasound applicator around on a patient, the user has to do this on the control device which is not always in reach, in which case the user has to move to the control device while still holding the ultrasound applicator, which is inconvenient.

The ultrasound therapy device known from the prior art also has the disadvantage that if a treatment with a different type of cup portion is desired, for example a larger cup portion for the application of ultrasound over a larger surface or a smaller cup portion for a more concentrated application of ultrasound over a smaller surface, then the entire ultrasound applicator has to be replaced. Hence, to be able to diversify the treatment possibilities multiple ultrasound applicators have to be provided which are provided with different types of cup portion, which takes up space and is more expensive.

### Disclosure of the invention

It is an aim of the present invention to provide an ultrasound therapy device with improved efficacy and safety in the treatment of a patient.

This aim is achieved according to the invention with an ultrasound therapy device showing the technical characteristics of the first independent claim, and with a computer implemented method for operating an ultrasound therapy device showing the technical characteristics of the second independent claim.

Therefore, the present invention provides, according to a first aspect, an ultrasound therapy device. The ultrasound therapy device comprises an ultrasound applicator. The ultrasound applicator comprises ultrasound generating means configured for generating ultrasound. The ultrasound applicator is configured for being held and moved by a user. The ultrasound therapy device comprises a motion detection system configured for detecting motion of the ultrasound applicator. Preferably, the motion detection system is arranged in the ultrasound applicator. The ultrasound therapy device comprises a processing unit operationally connected to the motion detection system. The processing unit is configured for receiving motion detection data from the motion detection system. The processing unit is configured for determining a translational velocity of the ultrasound applicator from the motion detection data. The processing unit is configured for generating at least one feedback signal based on the translational velocity of the ultrasound applicator. Preferably, the at least one feedback signal is configured for mitigating adverse effects and/or inefficacy of treatment with the ultrasound therapy device.

The ultrasound therapy device according to the first aspect of the present invention offers the advantage that the at least one feedback signal based on the translational velocity of the ultrasound applicator can be used to interact with a user of the ultrasound therapy device and/or to control the ultrasound therapy device in a way to improve the efficacy of the treatment and/or to prevent adverse effects of the treatment. For example, if the translational velocity of the ultrasound applicator detected by the motion detection system is too low the ultrasound therapy device could send as a feedback signal a warning to the user to increase the translational velocity of the ultrasound applicator, and/or the ultrasound therapy device could send as a feedback signal an instruction to the ultrasound generating means of the ultrasound applicator to decrease the intensity of the ultrasound generated by the ultrasound generating means, in order to prevent adverse effects on the patient, such as burns. If, on the other hand, the translational velocity of the ultrasound applicator detected by the motion detection system is too high the ultrasound therapy device could send as a feedback signal a signal to the user to decrease the translational velocity of the ultrasound applicator, and/or the ultrasound therapy device could send as a feedback signal an instruction to the ultrasound generating means of the ultrasound applicator to increase the intensity of the ultrasound generated by the ultrasound generating means, in order to improve the efficacy of the treatment.

In an embodiment of the ultrasound therapy device according to the present invention the processing unit is configured for generating the at least one feedback signal when the translational velocity of the ultrasound applicator is below a first threshold and/or when the translational velocity of the ultrasound applicator is above a second threshold. The first threshold is smaller than the second threshold.

This embodiment offers the advantage that an optimal range is defined for the translational velocity of the ultrasound application between the first threshold and the second threshold. In this optimal range a treatment with the ultrasound therapy device is performed in a safe and effective manner, and no feedback signal is necessarily required, but optionally at least one feedback signal could be generated for the purpose of informing the user of the ultrasound therapy device that the treatment with the ultrasound therapy device is being performed in the safe and effective optimal range. Outside this optimal range the treatment with the ultrasound therapy device is however no longer performed in a safe and effective manner, and the at least one feedback signal is required to inform the user of the ultrasound therapy device that the treatment with the ultrasound therapy device is being performed outside the safe and effective optimal range and/or to enable the ultrasound therapy device to mitigate adverse effects and/or inefficacy of the treatment outside the safe and effective optimal range. Hence, this embodiment further improves the efficacy and safety of the ultrasound therapy device.

In an embodiment of the ultrasound therapy device according to the present invention the processing unit is operationally connected to the ultrasound generating means. The processing unit is configured for generating a first feedback signal comprising instructions for the ultrasound generating means for setting the intensity of the ultrasound generated by the ultrasound generating means based on the translational velocity of the ultrasound applicator. The processing unit is configured for sending the first feedback signal to the ultrasound generating means for setting the intensity of the ultrasound generated by the ultrasound generating means according to the instructions in the first feedback signal.

This embodiment offers the advantage that the ultrasound therapy device itself is able to mitigate adverse effects and/or inefficacy of the treatment with the ultrasound therapy device without requiring interaction with the user of the ultrasound therapy device. Hence, this embodiment further improves the efficacy and safety of the ultrasound therapy device.

In an embodiment of the ultrasound therapy device according to the present invention the first feedback signal comprises instructions for the ultrasound generating means for decreasing the intensity of the ultrasound generated by the ultrasound generating means if the translational velocity of the ultrasound applicator is below the first threshold and/or for increasing the intensity of the ultrasound generated by the ultrasound generating means if the translational velocity of the ultrasound applicator is above the second threshold.

This embodiment offers the advantage that adverse effects of the treatment with the ultrasound therapy device can be mitigated by decreasing the intensity of the ultrasound generated by the ultrasound generating means when the translational velocity of the ultrasound applicator is below the first threshold, and/or that the inefficacy of the treatment with the ultrasound therapy device can be mitigated by increasing the intensity of the ultrasound generated by the ultrasound generating means when the translational velocity of the ultrasound applicator is above the second threshold. Hence, this embodiment further improves the efficacy and safety of the ultrasound therapy device.

In an embodiment of the ultrasound therapy device according to the present invention the first feedback signal comprises instructions for the ultrasound generating means for changing, preferably dynamically changing, the intensity of the ultrasound generated by the ultrasound generating means in correspondence with the change of the translational velocity of the ultrasound applicator.

This embodiment offers the advantage that adverse effects and/or inefficacy of the treatment with the ultrasound therapy device can be mitigated dynamically by the ultrasound therapy device in correspondence with the change of the translational velocity of the ultrasound applicator. Hence, this embodiment further improves the efficacy and safety of the ultrasound therapy device.

In an embodiment of the ultrasound therapy device according to the present invention the ultrasound therapy device comprises a user guiding system. The user guiding system comprises at least one indicator configured for indicating to a user to decrease and/or increase the translational velocity, i.e. to slow down and/or speed up the translational movement, of the ultrasound applicator. The processing unit is operatively connected to the user guiding system. The processing unit is configured for generating a second feedback signal comprising instructions for the at least one indicator for indicating to a user to decrease or increase the translational velocity of the ultrasound applicator based on the translational velocity of the ultrasound applicator. The processing unit is configured for sending the second feedback signal to the at least one indicator of the user guiding system for indicating to a user to decrease or increase the translational velocity of the ultrasound applicator according to the instructions in the second feedback signal.

This embodiment offers the advantage that the user can be guided by means of the user guiding system of the ultrasound therapy device to adapt the translational velocity or translational movement of the ultrasound applicator in such a way that the treatment with the ultrasound therapy device is safe and effective. Hence, this embodiment further improves the efficacy and safety of the ultrasound therapy device.

This embodiment also offers the advantage that the user guiding system makes the user aware of an incorrect use of the ultrasound therapy device for which the treatment with the ultrasound therapy device is unsafe and ineffective. As such, this embodiment of the ultrasound therapy device also forms a good training tool to learn the correct use of the ultrasound therapy device for which the treatment with the ultrasound therapy device is safe and effective.

In an embodiment of the ultrasound therapy device according to the present invention the at least indicator comprises at least one of a visual indicator, an auditive indicator, an haptic indicator and a textual indicator.

In an embodiment of the ultrasound therapy device according to the present invention the motion detection system comprises a 3-axis accelerometer. Preferably, the motion detection data comprises 3-axis acceleration data of the ultrasound applicator from the 3-axis accelerometer.

The 3-axis accelerometer is beneficial as a single sensor providing 3-axis acceleration data of the ultrasound applicator from which the three dimensional translational velocity of the ultrasound applicator can be determined with good accuracy by the processing unit.

In an embodiment of the ultrasound therapy device according to the present invention the motion detection system further comprises a 3-axis gyroscope. Preferably, the motion detection data comprises 3-axis angular orientation and/or angular velocity data of the ultrasound applicator from the 3-axis gyroscope. In an embodiment of the ultrasound therapy device according to the present invention the motion detection system further comprises a 3-axis magnetometer. Preferably, the motion detection data comprises 3-axis angular orientation data with respect to the geomagnetic field.

The 3-axis gyroscope and/or the 3-axis magnetometer are beneficial as further sensor or sensors in addition to the 3-axis accelerometer to respectively provide 3-axis angular orientation and/or angular velocity data and 3-axis angular orientation data with respect to the geomagnetic field to the processing unit, which data is combined by the processing unit with 3-axis acceleration data from the 3-axis accelerometer using sensor fusion to improve the accuracy of the three dimensional translational velocity of the ultrasound applicator determined by the processing unit.

The addition of the 3-axis gyroscope and/or the 3-axis magnetometer is also beneficial for distinguishing between a translational motion of the ultrasound applicator and a rotational motion of the ultrasound applicator with respect to a fixed point on the skin of a patient. In this way it can be prevented that such a rotational motion, for which there is a risk of adverse effects, is determined wrongly by the processing unit as a translational motion with a translational velocity in a safe range for which not even a feedback signal is generated or in a range for which a feedback signal is generated which is insufficient to prevent the adverse effects.

In an embodiment of the ultrasound therapy device according to the present invention the ultrasound applicator comprises user operable means configured for setting an intensity of the ultrasound generated by the ultrasound generating means.

The present invention also provides, according to a second aspect, an ultrasound therapy device. The ultrasound therapy device comprises an ultrasound applicator. The ultrasound applicator comprises ultrasound generating means configured for generating ultrasound. The ultrasound applicator is configured for being held and moved by a user. The ultrasound applicator comprises user operable means configured for setting an intensity of the ultrasound generated by the ultrasound generating means. Preferably, the ultrasound therapy device comprises a processing unit.

This embodiment and the ultrasound therapy device according to the second aspect of the present invention offer the advantage that a user of the ultrasound therapy device can set the intensity the ultrasound generated by the ultrasound generating means directly on the ultrasound applicator when performing ultrasound treatment on patient, and does not have to move to a control device of the ultrasound therapy device to change the intensity the ultrasound generated by the ultrasound generating means. This is beneficial for improving the conveniency or ease-of-use of the ultrasound therapy device.

In an embodiment of the ultrasound therapy device according to the present invention the processing unit is operationally connected to the ultrasound generating means and the user operable means. The processing unit is configured for receiving an instruction from the user operable means for setting the intensity of the ultrasound generated by the ultrasound generating means. The processing unit is configured for sending said instruction to the ultrasound generating means for setting the intensity of the ultrasound generated by the ultrasound generating means according to said instruction.

In an embodiment of the ultrasound therapy device according to the present invention the ultrasound therapy device comprises a control device configured for a user to view and change settings of the ultrasound therapy device, such as for example treatment parameters. The ultrasound applicator is connected to the control device, preferably at least for data communication and power delivery. The processing unit is arranged in the control device.

In an embodiment of the ultrasound therapy device according to the present invention the ultrasound applicator comprises a main body configured for being held and moved by a user. The ultrasound applicator comprises a cup portion housing the ultrasound generating means. The main body and the cup portion are configured for being removably connected to each other.

The present invention also provides, according to a third aspect, an ultrasound therapy device. The ultrasound therapy device comprises an ultrasound applicator. The ultrasound applicator comprises ultrasound generating means configured for generating ultrasound. The ultrasound applicator is configured for being held and moved by a user. The ultrasound applicator comprises a main body configured for being held and moved by a user. The ultrasound applicator comprises a cup portion housing the ultrasound generating means. The main body and the cup portion are configured for being removably connected to each other.

This embodiment and the ultrasound therapy device according to the third aspect of the present invention offer the advantage that the entire ultrasound applicator does not need to be replaced if treatment with a different type of cup portion is desired, for example a larger cup portion for the application of ultrasound over a larger surface or a smaller cup portion for a more concentrated application of ultrasound over a smaller surface. Only the cup portion itself has to be replaced on the main body of the ultrasound applicator. In this way a more versatile ultrasound therapy device is provided which can be used for different types of treatment, without needing a plurality of ultrasound applicators which take up space and which make the ultrasound therapy device more costly.

In an embodiment of the ultrasound therapy device according to the present invention the main body and the cup portion are configured for being removably connected to each other by means of a bayonet mount. The bayonet mount is configured such that the cup portion is connectable by means of rotation from any one of N start positions. N is at least two, preferably at most six, more preferably at most five, even more preferably at most four. A first element of the main body and the cup portion is provided with N connector sets for data and energy transmission. A second element of the main body and the cup portion, different from the first element, is provided with at least one connector set and at most N connector sets for data and energy transmission. The connector sets are positioned such that independent of the start position a connection for data and energy transmission is established between the main body and the cup portion when the main body and the cup portion are connected to each other.

This embodiment offers the advantage that the cup portion and the main body can be connected to each other by starting from any one of the start positions of the main body and the cup portion with respect to each other to establish a connection for data and energy transmission between main body and the cup portion. Hence, there is no need to start from a single predetermined start position to establish the connection for data and energy transmission, which is the case for bayonet mounts known from the prior art in which each of the bodies to be connected is only provided with a single connector set for data and energy transmission. Hence, this embodiment makes it easier to connect the main body and the cup portion of the ultrasound applicator to each other in the correct way to allow for data and energy transmission between the main body and the cup portion.

Furthermore, the present invention provides, according to the first aspect, a computer implemented method for operating an ultrasound therapy device. The ultrasound therapy device comprises an ultrasound applicator. The ultrasound applicator comprises ultrasound generating means configured for generating ultrasound. The ultrasound applicator is configured for being held and moved by a user. The ultrasound therapy device comprises a motion detection system configured for detecting motion of the ultrasound applicator. Preferably, the motion detection system is arranged in the ultrasound applicator. The ultrasound therapy device comprises a processing unit operationally connected to the motion detection system. The method comprises the step, performed by the processing unit, of receiving motion detection data from the motion detection system. The method comprises the step, performed by the processing unit, of determining a translational velocity of the ultrasound applicator from the motion detection data. The method comprises the step, performed by the processing unit, of generating at least one feedback signal based on the translational velocity of the ultrasound applicator.

In an embodiment of the computer-implemented method according to the present invention the at least one feedback signal is generated when the translational velocity of the ultrasound applicator is below a first threshold and/or when the translational velocity of the ultrasound applicator is above a second threshold. The first threshold is smaller than the second threshold.

In an embodiment of the computer-implemented method according to the present invention the processing unit is operationally connected to the ultrasound generating means. The method comprises the step, performed by the processing unit, of generating a first feedback signal comprising instructions for the ultrasound generating means for setting the intensity of the ultrasound generated by the ultrasound generating means based on the translational velocity of the ultrasound applicator. The method comprises the step, performed by the processing unit, of sending the first feedback signal to the ultrasound generating means for setting the intensity of the ultrasound generated by the ultrasound generating means according to the instructions in the first feedback signal.

In an embodiment of the computer-implemented method according to the present invention the first feedback signal comprises instructions for the ultrasound generating means for decreasing the intensity of the ultrasound generated by the ultrasound generating means if the translational velocity of the ultrasound applicator is below the first threshold and/or for increasing the intensity of the ultrasound generated by the ultrasound generating means if the translational velocity of the ultrasound applicator is above the second threshold.

In an embodiment of the computer-implemented method according to the present invention the first feedback signal comprises instructions for the ultrasound generating means for changing, preferably dynamically changing, the intensity of the ultrasound generated by the ultrasound generating means in correspondence with the change of the translational velocity of the ultrasound applicator.

In an embodiment of the computer-implemented method according to the present invention the ultrasound therapy device comprising a user guiding system. The user guiding system comprises at least one indicator configured for indicating to a user to decrease and/or increase the translational velocity, i.e. to slow down and/or speed up the translational movement, of the ultrasound applicator. The processing unit is operatively connected to the user guiding system. The method comprises the step, performed by the processing unit, of generating a second feedback signal comprising instructions for the at least one indicator for indicating to a user to decrease or increase the translational velocity of the ultrasound applicator based on the translational velocity of the ultrasound applicator. The method comprises the step, performed by the processing unit, of sending the second feedback signal to the at least one indicator of the user guiding system for indicating to a user to decrease or increase the translational velocity of the ultrasound applicator according to the instructions in the second feedback signal.

In an embodiment of the computer-implemented method according to the present invention the ultrasound applicator comprises user operable means configured for setting an intensity of the ultrasound generated by the ultrasound generating means. The processing unit is operationally connected to the ultrasound generating means and the user operable means. The method comprises the step, performed by the processing unit, of receiving an instruction from the user operable means for setting the intensity of the ultrasound generated by the ultrasound generating means. The method comprises the step, performed by the processing unit, of sending said instruction to the ultrasound generating means for setting the intensity of the ultrasound generated by the ultrasound generating means according to said instruction.

The present invention also provides, according to the second aspect, a computer implemented method for operating an ultrasound therapy device. The ultrasound therapy device comprises an ultrasound applicator. The ultrasound applicator comprises ultrasound generating means configured for generating ultrasound. The ultrasound applicator is configured for being held and moved by a user. The ultrasound applicator comprises user operable means configured for setting an intensity of the ultrasound generated by the ultrasound generating means. The ultrasound therapy device comprises a processing unit operationally connected to the ultrasound generating means and the user operable means. The method comprises the step, performed by the processing unit, of receiving an instruction from the user operable means for setting the intensity of the ultrasound generated by the ultrasound generating means. The method comprises the step, performed by the processing unit, of sending said instruction to the ultrasound generating means for setting the intensity of the ultrasound generated by the ultrasound generating means according to said instruction.

Each of the first aspect, the second aspect and the third aspect of the present invention can be implemented independently of the other aspects of the present invention. Each of the first aspect, the second aspect and the third aspect of the present invention can also be implemented in combination with any one of the other aspects of the present invention.

### Brief description of the drawings

The invention will be further elucidated by means of the following description and the appended figures.
Figure 1 shows a perspective view from above on an ultrasound therapy device according to an embodiment of the present invention with an ultrasound applicator having a replaceable cup portion.
Figure 2 shows a perspective view from below on an ultrasound applicator according to an embodiment of the present invention with a fixed cup portion.
Figure 3 shows a perspective view from above on the ultrasound applicator of Figure 2 arranged in a holder.
Figure 4 shows a perspective view from above on an ultrasound applicator according to an embodiment of the present invention with a first type of cup portion connected to the main body.
Figure 5 shows a perspective view from above on the ultrasound applicator of Figure 4 with a second type of cup portion connected to the main body.
Figure 6 shows a perspective view from below on the ultrasound applicator of Figure 4 or Figure 5 without a cup portion connected to the main body.
Figure 7 shows a perspective view from above on the second type of cup portion of Figure 5.
Figure 8 shows a holder for an ultrasound applicator according to an embodiment of the present invention.
Figure 9 shows a schematic representation of an ultrasound therapy device according to an embodiment of the present invention.

### Modes for carrying out the invention

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

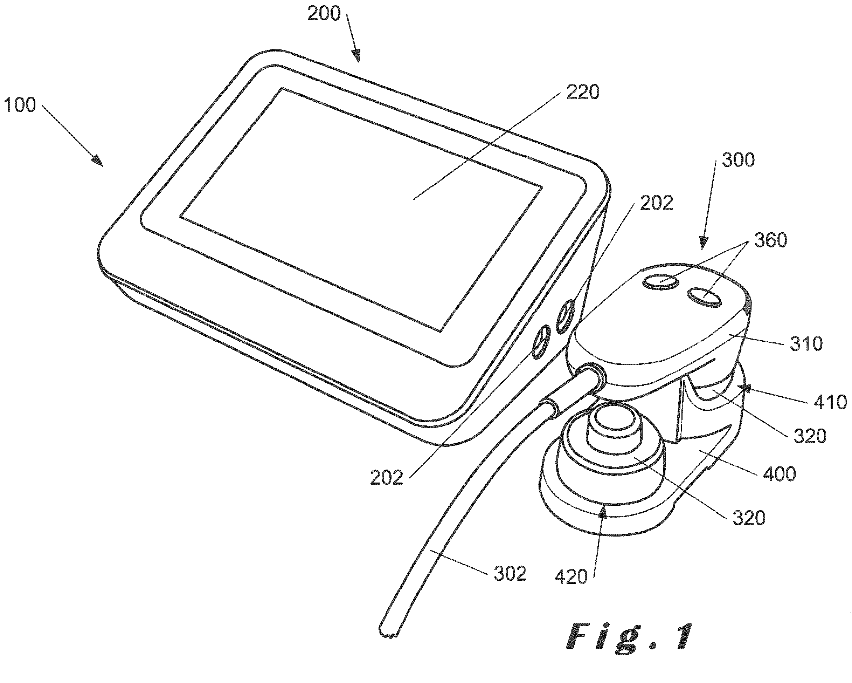

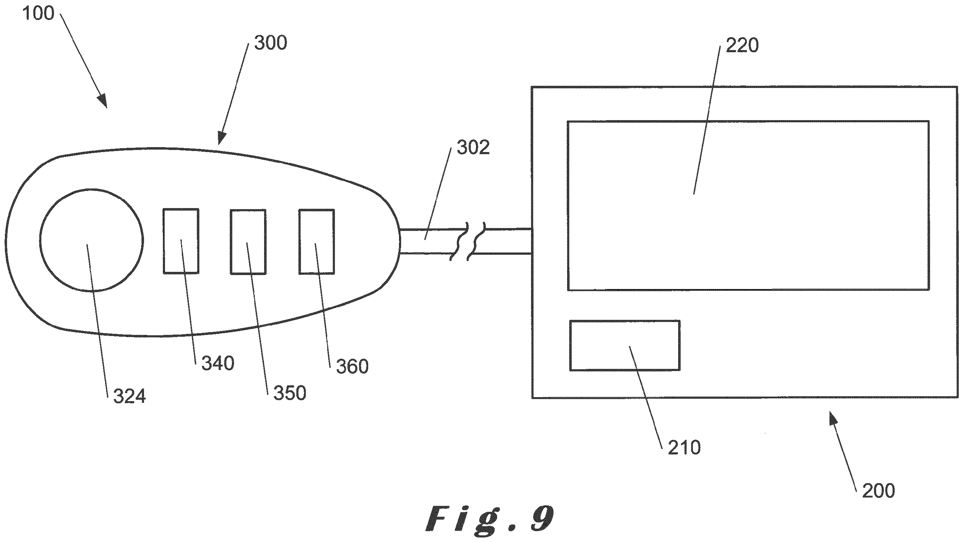

Figure 1 shows a perspective view on an ultrasound therapy device 100 according to an embodiment of the present invention. A schematic representation of an ultrasound therapy device 100 according to the present invention is shown in Figure 9.

The ultrasound therapy device 100 comprises a control device 200. The control device 200 is provided with a touch screen 220 via which a user can interact with the ultrasound therapy device 100 for viewing and changing settings of the ultrasound therapy device 100. For the purpose of powering the ultrasound therapy device 100 the control device 200 is provided for being connected to a power source, such as for example the power grid, by means of a power cord, or is provided with a power source, such as for example a battery.

The ultrasound therapy device 100 further comprises a processing unit 210 which is configured for controlling the ultrasound therapy device 100. Preferably, the processing unit 210 is arranged in the control device 200 of the ultrasound therapy device 100, as shown in Figure 9.

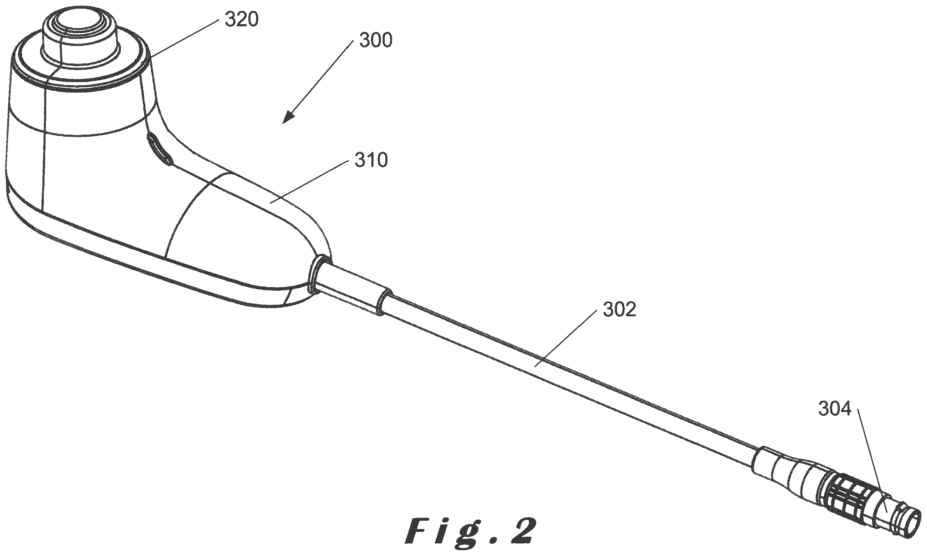

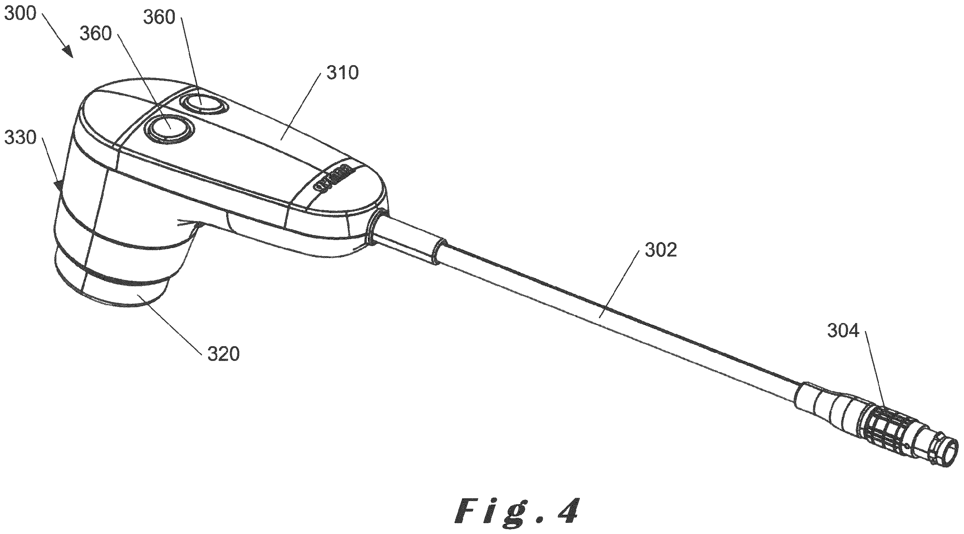

The ultrasound therapy device 100 further comprises an ultrasound applicator 300, of which a first embodiment is shown in Figures 2-3 and a second embodiment is shown in Figures 4-7. It should be noted that the ultrasound therapy device 10 shown in Figure 1 is provided with an ultrasound applicator 300 according the second embodiment of the present invention.

The ultrasound applicator 300 is provided with a cord 302 and a plug 304 by means of which the ultrasound applicator 300 is to be connected to the control device 200 via any one of the sockets 202 on the control device 200. This connection is provided for energy and data transmission between the ultrasound therapy device 100.

The ultrasound applicator 300 comprises a main body 310 which is configured for being held and moved by a user of the ultrasound therapy device 100. This allows the user to hold the ultrasound applicator 300 and move the ultrasound applicator 300 over the body of a patient, more specifically over the skin of the patient, for applying a therapeutic ultrasound treatment.

The ultrasound applicator 300 further comprises a cup portion 320. The cup portion 320 houses an ultrasound generating means 324 of the ultrasound applicator 300, which can be seen in Figure 9. The ultrasound generating means 324 are configured for generating ultrasound, i.e. ultrasound to be applied to a patient when the ultrasound applicator 300 is moved over the body of the patient during a therapeutic ultrasound treatment. The ultrasound generating means 324 are operationally connected to the processing unit 210 of the ultrasound therapy device 100, such that the processing unit 210 can at least activate and deactivate the ultrasound generating means 324, and preferably also set the intensity of the ultrasound generated by the ultrasound generating means 324.

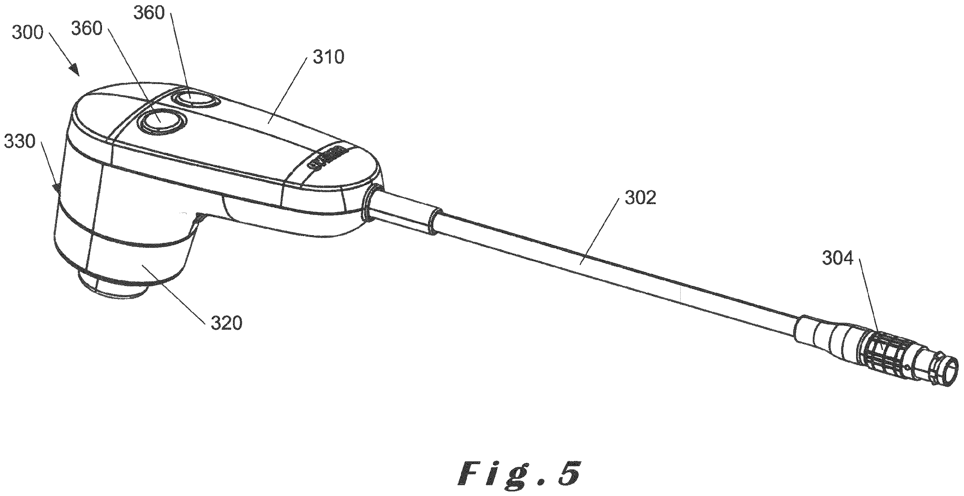

In the ultrasound applicator 300 according to the first embodiment the cup portion 320 is attached in a fixed manner to the main body 310 of the ultrasound applicator 300. In the ultrasound applicator 300 according to the second embodiment the cup portion 310 is removably connected to the main body 310 of the ultrasound applicator 300. With the ultrasound applicator 300 according to the second embodiment the cup portion 320 on the ultrasound applicator 300 can be replaced easily with a cup portion 320 of a different type if a treatment with a different type of cup portion 320 is desired. Different types of cup portion 320 could for example be a larger cup portion 320, such as shown in Figure 4, for the application of ultrasound over a larger surface or a smaller cup portion 320, such as shown in Figure 5, for a more concentrated application of ultrasound over a smaller surface. Hence, with the ultrasound applicator 300 according to the second embodiment there is no need to connect an entire different ultrasound applicator 300 to the control device 200 if a treatment with a different type of cup portion 320 is desired. Further details of the connection mechanism between the main body 310 and the cup portion 320 of the ultrasound applicator 300 according to the second embodiment will be explained in further detail below.

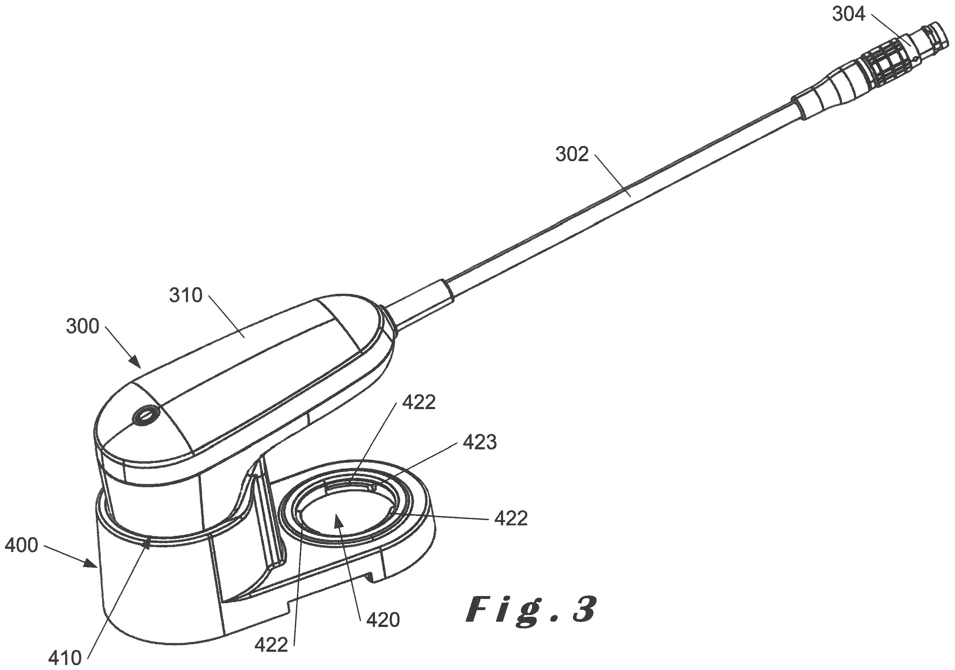

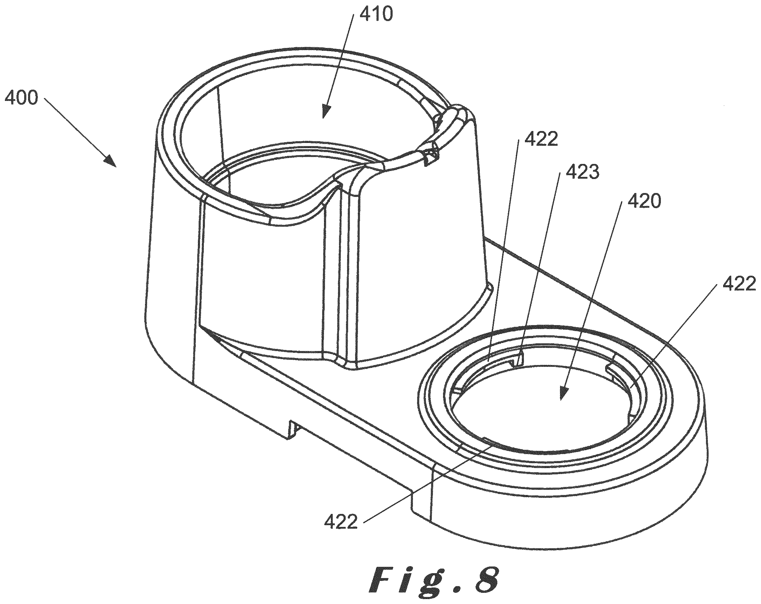

The ultrasound therapy device 100 further comprises a holder 400, as can be seen in Figures 1, 3 and 8. The holder 400, shown in detail in Figure 8, comprises a first recessed portion 410 which is configured for receiving a front part of the ultrasound applicator 300 and thereby supporting the ultrasound applicator 300, such as illustrated in Figures 1 and 3. In this way the ultrasound applicator 300 can be stored away safely when the ultrasound applicator 300 is not being used. The holder 400 comprising a second recessed portion 420 which is configured for partially receiving a separate cup portion 320 of the ultrasound applicator 300 according to the second embodiment, such as illustrated in Figure 1. In this way a cup portion 320 that is not connected to the main body 310 of the ultrasound applicator 300, and that is thus not in use, can be stored away safely.

The ultrasound therapy device 100 further comprises a motion detection system 340 configured for detecting the motion of the ultrasound applicator 300, and for generating motion detection data relating to the detected motion of the ultrasound applicator 300. Preferably, the motion detection system 340 is arranged in the ultrasound applicator 300, as can be seen in Figure 9.

Preferably, the motion detection system 340 comprises at least a 3-axis accelerometer. The 3-axis accelerometer generates 3-axis acceleration data of the ultrasound applicator 300 as part of the motion detection data. The 3-axis acceleration data from the 3-axis accelerometer is beneficial for determining therefrom a translational velocity of the ultrasound applicator 300, more specifically a 3-axis velocity of the ultrasound applicator 300 with which the ultrasound applicator 300 is translated along a trajectory through a 3D-space.

Preferably, the motion detection system 340 comprises, in addition to the 3-axis accelerometer, at least one of a 3-axis gyroscope and a 3-axis magnetometer. The 3-axis gyroscope generates 3-axis angular orientation and/or angular velocity data of the ultrasound applicator 300 as part of the motion detection data. The 3-axis magnetometer generates 3-axis angular orientation data with respect to the geomagnetic field of the ultrasound applicator 300 as part of the motion detection data. The 3-axis angular orientation and/or angular velocity data from the 3-axis gyroscope and/or the 3-axis angular orientation data with respect to the geomagnetic field from the 3-axis magnetometer is beneficial for being combined with the 3-axis acceleration data from the 3-axis accelerometer using sensor fusion in order to determine a translational velocity of the ultrasound applicator 300 with improved accuracy. The addition of the 3-axis angular orientation and/or angular velocity data from the 3-axis gyroscope and/or the 3-axis angular orientation data with respect to the geomagnetic field from the 3-axis magnetometer is also beneficial for distinguishing between a translational motion of the ultrasound applicator and a rotational motion of the ultrasound applicator 300.

The processing unit 210 of the ultrasound therapy device 100 is operationally connected to the motion detection system 340, and configured for receiving the motion detection data from the motion detection system 340, for determining the translational velocity of the ultrasound applicator 300 from the motion detection data, and for generating at least one feedback signal based on the determined translational velocity of the ultrasound applicator 300. The at least one feedback signal may for example be used in the ultrasound therapy device itself or provided to a user of the ultrasound therapy device 100, for the purpose of ensuring a safe and effective treatment of a patient with the ultrasound therapy device 100.

Preferably, the processing unit 210 is configured for at least generating the at least one feedback signal when the translational velocity of the ultrasound applicator 300 is below a first threshold, below which first threshold the ultrasound applicator 300 is being moved too slow on a patient by a user, and there is a risk for adverse effects for the patient such as internal burns. Preferably, the processing unit 210 is configured for at least generating the at least one feedback signal when the translational velocity of the ultrasound applicator 300 is above a second threshold, which second threshold is larger than the first threshold, and above which second threshold the ultrasound applicator 300 is being moved too fast on a patient by user, and the treatment of the patient with the ultrasound therapy device 100 is no longer effective. Such a first threshold and second threshold are usually known to the skilled person for a given type of ultrasound generating means 324, or may be derived empirically by the skilled person. The first threshold and/or the second threshold may be dependent on the intensity of the ultrasound generated by the ultrasound generating means 324. Optionally, the processing unit 210 may also be configured for generating the at least one feedback signal when the translational velocity of the ultrasound applicator 300 is between or equal to the first threshold and the second threshold, for example for informing a user of the ultrasound therapy device 100 that a treatment of a patient with the ultrasound therapy device 100 is being performed in a safe and effective manner.

The at least one feedback signal may comprise a first feedback signal. The first feedback signal comprises instructions for the ultrasound generating means 324 for setting the intensity of the ultrasound generated by the ultrasound generating means 324 based on the translational velocity of the ultrasound applicator 300. Preferably, the first feedback signal comprises instructions for the ultrasound generating means 324 for changing the intensity of the ultrasound generated by the ultrasound generating means 324 in correspondence with or proportional to the change of the translational velocity of the ultrasound applicator 300. Preferably, the first feedback signal comprises instructions for the ultrasound generating means 324 for decreasing the intensity of the ultrasound generated by the ultrasound generating means 324 if the translational velocity of the ultrasound applicator 300 is below the first threshold and/or for increasing the intensity of the ultrasound generated by the ultrasound generating means 324 if the translational velocity of the ultrasound applicator 300 is above the second threshold. The processing unit 210 is configured for sending the first feedback signal to the ultrasound generating means 324 for setting the intensity of the ultrasound generated by the ultrasound generating means 324 according to the instructions in the first feedback signal.

The ultrasound therapy device 100 further comprises a user guiding system 350. The user guiding system 350 comprises at least one indicator configured for indicating to a user of the ultrasound therapy device 100 to decrease and/or increase the translational velocity of the ultrasound applicator 300. The user guiding system 350 may be arranged in the ultrasound applicator 300, as can be seen in Figure 9, but may also be incorporated in other component of the ultrasound therapy device 100, or be a separate component of the ultrasound therapy device 100. The at least indicator of the user guiding system 350 may for example comprise a visual indicator, such as a LED light, an auditive indicator, such as a speaker, an haptic indicator and/or a textual indicator, such as a screen configured for displaying a text message. For use with the guiding system 350, the at least one feedback signal generated by the processing unit may comprise a second feedback signal. The second feedback signal comprises instructions for the at least one indicator for indicating to a user to decrease or increase the translational velocity of the ultrasound applicator 300 based on the translational velocity of the ultrasound applicator 300. Preferably, the second feedback signal comprises instructions for the at least one indicator for indicating to a user to decrease the translational velocity of the ultrasound applicator 300 if the translational velocity of the ultrasound applicator 300 is below the first threshold and/or to increase the translational velocity of the ultrasound applicator 300 if the translational velocity of the ultrasound applicator 300 is above the second threshold.

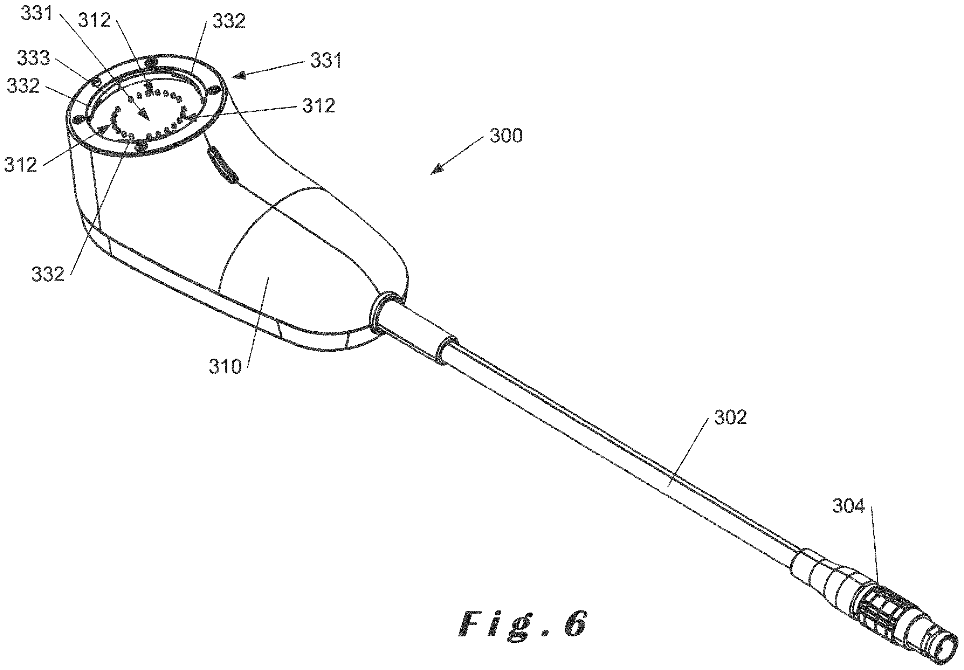

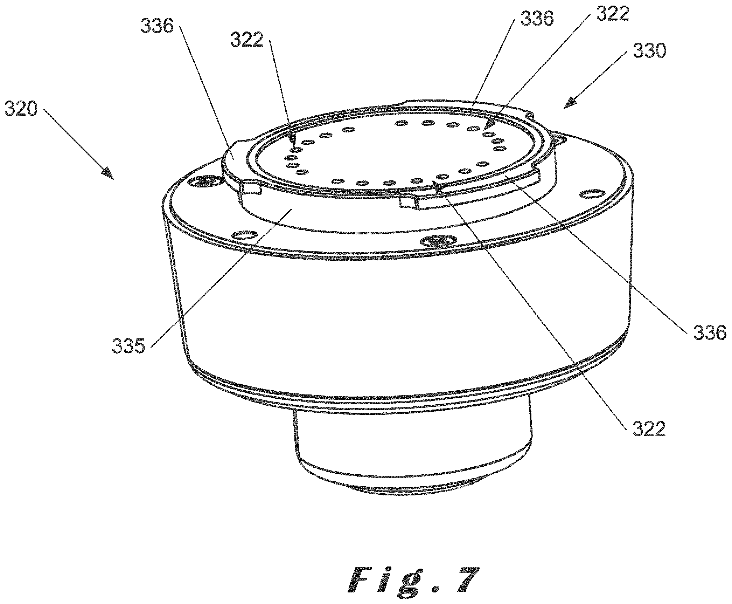

In the ultrasound applicator 300 according to the second embodiment the main body 310 and the cup portion 320 to are configured for being removably connected to each other by means of a bayonet mount 330, as illustrated in Figures 6 and 7. Therefore, the main body 310 is provided with a circular recessed portion 331. The cup portion 320 is provided with a circular protruding portion 335 configured for being received in the recessed portion 331 of the main body 310 for establishing the connection between the cup portion 320 and the main body 310.

The recessed portion 331 of the main body 310 is provided along the inner circumference with three inwards protruding wings 332. The wings 332 are of equal length, are uniformly distributed along the inner circumference of the recessed portion 331 and are provided at corresponding ends with a downwards extending stop 333. At the bottom the recessed portion 331 is provided with three connector sets 312 for data and energy transmission. Each connector set 312 comprises seven connectors. In alternative embodiments the connector sets 312 may however comprise more or less connectors, for example depending on the requirements for the data and energy transmission.

The protruding portion 335 of the cup portion 320 is provided along the outer circumference with three outwards protruding wings 336. The wings 336 are of equal length and are uniformly distributed along the outer circumference of the protruding portion 335. At the top the protruding portion 335 is provided with three connector sets 322 for data and energy transmission. Each connector set 322 comprises seven connectors, corresponding to the number of connectors in the connector sets 312 on the recessed portion 331 of the main body 310. In alternative embodiments the connector sets 322, such as the connector sets 312 on the recessed portion 331 of the main body 310, may however comprise more or less connectors, for example depending on the requirements for the data and energy transmission.

The wings 336 on the protruding portion 335 of the cup portion 320 and the wings 332 in the recessed portion 331 of the main body 310 are arranged such that the wings 336 on the protruding portion 335 fit between the open spaces between the wings 332 in the recessed portion 331.

For connecting the main body 310 and cup portion 320 with each other the main body 310 and the cup portion 320 are positioned close to each other with the protruding portion 335 on the cup portion 320 and the recessed portion 331 in the main body 310 facing each other in any one of three possible start positions in which the wings 336 on the protruding portion 335 are aligned with the open spaces between the wings 332 in the recessed portion 331. Then, the main body 310 and the cup portion 320 are moved towards each other to insert the protruding portion 336 on the cup portion 320 into the recessed portion 331 in the main body 310. Thereby, the wings 336 on the protruding portion 335 move between the wings 332 in the recessed portion 331 until the wings 336 on the protruding portion 335 are situated below the level of the wings 332 in the recessed portion 331. Then, the main body 310 and the cup portion 320 are rotated with respect to each other. Thereby, the wings 336 on the protruding portion 335 rotate below the wings 332 in the recessed portion 331 until the wings 336 on the protruding portion 335 are stopped against the stops 333 on the wings 332 in the recessed portion 331. In this position, the connection between the main body 310 and the cup portion 320 is established. In this position, each connector set 312 on the main body 310 is also aligned and in contact with a corresponding connector set 322 on the cup portion 310, such that also a connection for data and energy transmission is established between the main body 310 and the cup portion 320.

Each of the main body 310 and the cup portion 320 of the ultrasound applicator 300 being provided with the three connector sets 312, 322, as described above, offers the advantage that, independent of the start position of the cup portion 320 and the main body 310 with respect to each other, a connection for data and energy transmission between the main body 310 and the cup portion 320 is established when the cup portion 320 and the main body 310 are connected to each other. It should be noted that in alternative embodiments of the ultrasound applicator 300 the same effect may be achieved if either one of the main body 310 and the cup portion 320 is provided with one or two of the connector sets 312, 322 and the other one of the main body 310 and the cup portion 320 with the three connector sets 310, 320. In such case there would always be established a connection for data and energy transmission between at least one pair of corresponding connector sets 310, 320 on the main body 310 and the cup portion 320.

Similarly to the recessed portion 331 in the main body 310 of the ultrasound applicator 300, the second recessed portion 420 in the holder 400 for the ultrasound applicator 300 is also provided along the inner circumference with three inwards protruding wings 422 having stops 423, as can be seen in Figure 8, to be able to connect a cup portion 320 of the ultrasound applicator 300 to the holder 400 by means of a bayonet mount.

In alternative embodiments of the ultrasound applicator 300 the number N of wings 332, 336 in the recessed portion 331 of the main body 310 and on the protruding portion 335 of the cup portion 320 can be different from three, but should be at least two, and is preferably at most six, more preferably at most five and even more preferably at most four. Hereby, either one of the main body 310 and the cup portion 320 should be provided with the same number N of connector sets 312, 322 and the other one of the main body 310 and the cup portion 320 should be provided with any number of one to N connector sets 310, 320. Hereby, the second recessed portion 420 in the holder 400 for the ultrasound applicator should also be provided with a corresponding number N of wings 422.

In alternative embodiments of the ultrasound applicator 300 the recessed portion 331 and the protruding portion 335, as described above, may also be provided on respectively the cup portion 320 and the main body 310. Hereby, the second recessed portion 420 in the holder 400 for the ultrasound applicator 300 should be replaced with a protruding portion similar to the protruding portion 335 on the main body 310 of the ultrasound applicator 300 in such case.

The ultrasound applicator 300 according to the second embodiment is also provided with two buttons 360, which form user operable means 360 that allow a user holding the ultrasound applicator 300 to directly set an intensity of the ultrasound generated by the ultrasound generating means 324 on the ultrasound applicator 300, instead of having to do this on the control device 200. For this purpose, the processing unit 210 of the ultrasound therapy device 100 is operationally connected to the user operable means 360, and also to the ultrasound generating means 324 as already explained above. The processing unit 210 is thereby configured for receiving an instruction from the user operable means 360 for setting the intensity of the ultrasound generated by the ultrasound generating means 324, and for sending said instruction to the ultrasound generating means 324 for setting the intensity of the ultrasound generated by the ultrasound generating means 324 according to said instruction.

In this case, one of the buttons 360 is used for increasing the intensity of the ultrasound generated by the ultrasound generating means 324, and the other one of the buttons 360 is used for decreasing the intensity of the ultrasound generated by the ultrasound generating means 324. It should be noted that in alternative embodiments of the ultrasound therapy device 100 any other suitable type of user operable means 360 known to the skilled person may be used which allows for setting the intensity of the ultrasound generated by the ultrasound generating means 324. It should also be noted that the ultrasound applicator 300 according to the first embodiment may also be provided with such ultrasound generating means 360.

## Claims

1. An ultrasound therapy device (100), wherein the ultrasound therapy device (100) comprises:
an ultrasound applicator (300), wherein the ultrasound applicator (300) comprises ultrasound generating means (324) configured for generating ultrasound, wherein the ultrasound applicator (300) is configured for being held and moved by a user,
**characterised in that** the ultrasound therapy device (100) further comprises:
a motion detection system (340) configured for detecting motion of the ultrasound applicator (300); and
a processing unit (210) operationally connected to the motion detection system (340), wherein the processing unit (210) is configured for receiving motion detection data from the motion detection system (340), wherein the processing unit (210) is configured for determining a translational velocity of the ultrasound applicator (300) from the motion detection data, and wherein the processing unit (210) is configured for generating at least one feedback signal based on the translational velocity of the ultrasound applicator (300).

2. The ultrasound therapy device (100) according to claim 1, wherein the processing unit (210) is configured for generating the at least one feedback signal when the translational velocity of the ultrasound applicator (300) is below a first threshold and/or when the translational velocity of the ultrasound applicator (300) is above a second threshold, wherein the first threshold is smaller than the second threshold.

3. The ultrasound therapy device (100) according to claim 1 or 2, wherein the processing unit (210) is operationally connected to the ultrasound generating means (324), wherein the processing unit (210) is configured for generating a first feedback signal comprising instructions for the ultrasound generating means (324) for setting the intensity of the ultrasound generated by the ultrasound generating means (324) based on the translational velocity of the ultrasound applicator (300), wherein the processing unit (210) is configured for sending the first feedback signal to the ultrasound generating means (324) for setting the intensity of the ultrasound generated by the ultrasound generating means (324) according to the instructions in the first feedback signal.

4. The ultrasound therapy device (100) according to claim 3, at least in combination with claim 2, wherein the first feedback signal comprises instructions for the ultrasound generating means (324) for decreasing the intensity of the ultrasound generated by the ultrasound generating means (324) if the translational velocity of the ultrasound applicator (300) is below the first threshold and/or for increasing the intensity of the ultrasound generated by the ultrasound generating means (324) if the translational velocity of the ultrasound applicator (300) is above the second threshold.

5. The ultrasound therapy device (100) according to claim 3 or 4, wherein the first feedback signal comprises instructions for the ultrasound generating means (324) for changing the intensity of the ultrasound generated by the ultrasound generating means (324) in correspondence with the change of the translational velocity of the ultrasound applicator (300).

6. The ultrasound therapy device (100) according to any one of the claims 1-5, wherein the ultrasound therapy device (100) comprises a user guiding system (350), wherein the user guiding system (350) comprises at least one indicator configured for indicating to a user to decrease and/or increase the translational velocity of the ultrasound applicator (300), wherein the processing unit (210) is operatively connected to the user guiding system (350), wherein the processing unit (210) is configured for generating a second feedback signal comprising instructions for the at least one indicator for indicating to a user to decrease or increase the translational velocity of the ultrasound applicator (300) based on the translational velocity of the ultrasound applicator (300), wherein the processing unit (210) is configured for sending the second feedback signal to the at least one indicator of the user guiding system (350) for indicating to a user to decrease or increase the translational velocity of the ultrasound applicator (300) according to the instructions in the second feedback signal.

7. The ultrasound therapy device (100) according to claim 6, wherein the at least indicator comprises at least one of a visual indicator, an auditive indicator, an haptic indicator and a textual indicator.

8. The ultrasound therapy device (100) according to any one of the claims 1-7, wherein the motion detection system (340) comprises a 3-axis accelerometer.

9. The ultrasound therapy device (100) according to claim 8, wherein the motion detection system (340) further comprises a 3-axis gyroscope, and/or wherein the motion detection system (340) further comprises a 3-axis magnetometer.

10. The ultrasound therapy device (100) according to any one of the claims 1-9, wherein the ultrasound applicator (300) comprises user operable means (360) configured for setting an intensity of the ultrasound generated by the ultrasound generating means (324).

11. The ultrasound therapy device (100) according to claim 10, wherein the processing unit (210) is operationally connected to the ultrasound generating means (324) and the user operable means (360), wherein the processing unit (210) is configured for receiving an instruction from the user operable means (360) for setting the intensity of the ultrasound generated by the ultrasound generating means (324), and wherein the processing unit (210) is configured for sending said instruction to the ultrasound generating means (324) for setting the intensity of the ultrasound generated by the ultrasound generating means (324) according to said instruction.

12. The ultrasound therapy device (100) according to any one of the claims 1-11, wherein the ultrasound therapy device (100) comprises a control device (200) configured for a user to view and change settings of the ultrasound therapy device (100), wherein the ultrasound applicator (300) is connected to the control device (200), wherein the processing unit (210) is arranged in the control device (200).

13. The ultrasound therapy device (100) according to any one of the claims 1-12, wherein the ultrasound applicator (300) comprises:
a main body (310) configured for being held and moved by a user; and
a cup portion (320) housing the ultrasound generating means (324),
wherein the main body (310) and the cup portion (320) are configured for being removably connected to each other.

14. The ultrasound therapy device (100) according to claim 13, wherein the main body (310) and the cup portion (320) are configured for being removably connected to each other by means of a bayonet mount (330), wherein the bayonet mount (330) is configured such that the cup portion (320) is connectable by means of rotation from any one of N start positions, wherein N is at least two, preferably at most six, more preferably at most five, even more preferably at most four, wherein a first element of the main body (310) and the cup portion (320) is provided with N connector sets (312, 322) for data and energy transmission, wherein a second element of the main body (310) and the cup portion (320) different from the first element is provided with at least one connector set (312, 322) and at most N connector sets (312, 322) for data and energy transmission, wherein the connector sets (312, 322) are positioned such that independent of the start position a connection for data and energy transmission is established between the main body (310) and the cup portion (320) when the main body (310) and the cup portion (320) are connected to each other.

15. A computer implemented method for operating an ultrasound therapy device (100), wherein the ultrasound therapy device (100) comprises:
an ultrasound applicator (300), wherein the ultrasound applicator (300) comprises ultrasound generating means (324) configured for generating ultrasound, wherein the ultrasound applicator (300) is configured for being held and moved by a user;
a motion detection system (340) configured for detecting motion of the ultrasound applicator (300); and
a processing unit (210) operationally connected to the motion detection system (340),
wherein the method comprises the steps, performed by the processing unit (210), of:
receiving motion detection data from the motion detection system (340);
determining a translational velocity of the ultrasound applicator (300) from the motion detection data; and
generating at least one feedback signal based on the translational velocity of the ultrasound applicator (300).
